## Europäisches Patentamt
## European Patent Office
## Office européen des brevets

(19)

(11) Veröffentlichungsnummer: **0 002 691**
**B1**

(12) **EUROPÄISCHE PATENTSCHRIFT**

(45) Veröffentlichungstag der Patentschrift: **03.06.81**

(21) Anmeldenummer: **78101578.9**

(22) Anmeldetag: **06.12.78**

(51) Int. Cl.³: **C 07 C 143/60**

(54) Verfahren zur Herstellung von 1-Aminonaphthalin-3,6,8-trisulfonsäure.

(30) Priorität: **22.12.77 DE 2757412**

(43) Veröffentlichungstag der Anmeldung:
**11.07.79 Patentblatt 79/14**

(45) Bekanntmachung des Hinweises auf die Patenterteilung:
**03.06.81 Patentblatt 81/22**

(84) Benannte Vertragsstaaten:
**CH DE FR GB**

(56) Entgegenhaltungen:
**DE - A - 2 425 811**
**DE - B - 1 233 408**
**FR - A - 550 850**

**CHEMICAL ABSTRACTS, Vol. 82, Nr. 23, 9. Juni 1975, Zusammenfassung Nr. 155962g, Columbus, Ohio, USA**
**IKEDA TAKUO et al., "Naphthylaminesulfonic acids", Seite 574, linke Spalte**

**CHEMICAL ABSTRACTS, Vol. 84, Nr. 7 1976, Zusammenfassung Nr. 43703h, Columbus, Ohio, USA**
**YAMAMOTO TADASHI et al., "Naphthylamine-sulfonic acids", Seite 459, linke Spalte**

(73) Patentinhaber: **BAYER AG**
**Zentralbereich Patente, Marken und Lizenzen**
**D-5090 Leverkusen 1, Bayerwerk (DE)**

(72) Erfinder: **Braden, Rudolf, Dr.**
**Nothauser Feld 1**
**D-5068 Odenthal (DE)**
Erfinder: **Behre, Horst, Dr.**
**Im Hellsiefen 4**
**D-5068 Odenthal (DE)**
Erfinder: **Ziemann, Heinz, Dr.**
**Am Wiessenberg 10**
**D-5653 Leichlingen (DE)**

# 0 002 691

## Verfahren zur Herstellung von 1-Aminonaphthalin-3,6,8-trisulfonsäure

Die vorliegende Erfindung betrifft ein Verfahren zur Herstellung der 1-Aminonaphthalin-3,6,8-trisulfonsäure (Koch'sche Säure, T-Säure) als Salz aus einem Salz der 8-Nitro-naphthalin-1,3,6-trisulfonsäure durch katalytische Hydrierung.

Die 1-Amino-naphthalin-3,6,8-trisulfonsäure, im folgenden auch als T-Säure bezeichnet, ist das Vorprodukt für 1-Amino-8-naphthol-3,6-disulfonsäure (H-Säure). Die H-Säure ist ein bedeutendes Zwischenprodukt für die Herstellung von Farbstoffen (s. Ullmanns Enzyklopädie der Technischen Chemie, 3. Auflage, 12. Band, S. 621).

Die Herstellung der 1-Amino-naphthalin-3,6,8-trisulfonsäure (T-Säure) als Calcium-Natriumsalz durch Reduktion der Salze der 8-Nitronaphthalin-1,3,6-trisulfonsäure ist bekannt (s. Kirk-Othmer, Encyclopedia of Chem. Technology Vol. 13, S. 716, 1967). Häufig wurde nach Béchamp mit Eisenpulver in Gegenwart von Essigsäure reduziert (s. Fiat Final Report Nr. 1016, S. 38. 1945). Die sich bildenden gelösten Eisensalze werden durch Zugabe von Magnesiumoxid gefällt. Es entsteht ein Eisenoxidschlamm, der abgetrennt wird. Durch Zugabe von Steinsalz und Salzsäure wird dann die T-Säure als saures Salz ausgefällt und abfiltriert. In dem Filtrat verbleiben neben den Chloriden und Sulfaten Acetat- und Magnesium-Salze von beim Reduktionsschritt zugesetzter Essigsäure und nach dem Reduktionsschritt zugesetztem Magnesiumoxid.

Die Reduktion der Salze der 8-Nitro-naphthalin-1,3,6-trisulfonsäure nach Béchamp hat für die Technik erhebliche Nachteile: Die Zugabe relativ großer Mengen Eisenpulver und mehr noch die Abtrennung des entstehenden Eisenoxidschlammes sind Operationen, die einem kontinuierlichen großtechnischen Verfahren hinderlich sind. Der anfallende Eisenoxidschlamm, der neben Alkali- und Erdalkalisalzen noch organische Bestandteile enthält, ist nur in technisch schwieriger und unwirtschaftlicher Weise aufzuarbeiten. Die für die Reduktion erforderliche Essigsäure und das zu deren Neutralisation eingesetzte Magnesiumoxid führen zu erheblichen Mengen an Salzen im Filtrat, die nur äußerst schwierig abzutrennen sind. Daher besteht seit langem der Bedarf nach einem fortschrittlichen Verfahren, das diese gravierenden Nachteile vermeidet.

Weitere Verfahren zur Reduktion von 1-Nitro-naphthalin-3,6,8-trisulfonsäure sind in C.A. *82*, 155962g und C.A. *84*, 43703h beschrieben. Gemäß diesen Verfahren wird 1-Nitro-naphthalin-3,6,8-trisulfonsäure mit Wasserstoff in Gegenwart von Raney-Nickel bzw. Pd-C-Katalysatoren reduziert. Nachteilig an diesen Verfahren ist, daß sie 1-Aminonaphthalin-3,6,8-trisulfonsäure nur in Ausbeuten von etwa 80% liefern.

Weiterhin war bekannt, daß man aromatische Nitroverbindungen in Gegenwart von Sulfiden von Metallen der 8. Gruppe des Periodensystems der Elemente und von Rhenium katalytisch zu aromatischen Aminen reduzieren kann, beispielsweise 4,4'-Dinitrostilben-2,2'-disulfonsäure zu 4,4'-Diaminostilben-2,2'-disulfonsäure (s. DT—OS 2 425 811).

Da jedoch, wie aus den in C.A. *82*, 155962g und C.A. *84*, 43703h beschriebenen Verfahren hervorgeht, der Ersatz des für die katalytische Reduktion von Nitrogruppen anerkannt guten Raney-Nickels durch den für die katalytische Reduktion von Nitrogruppen ebenfalls anerkannt guten Katalysator Pd-C unter den angewendeten Reaktionsbedingungen, d.h. in alkalischen Medien, keine Verbesserung der Ausbeuten an 1-Aminonaphthalin-3,6,8-trisulfonsäure bringt, war nicht zu erwarten, daß mit den der DT—OS 24 25 811 für die Reduktion von Nitrogruppen beschriebenen Katalysatoren unter den gleichen Reaktionsbedingungen verbesserte Ausbeuten erreichbar sein würden.

Es wurde nun ein Verfahren zur Herstellung von 1-Aminonaphthalin-3,6,8-trisulfonsäure durch Reduktion der alkalisch-wäßrigen Lösungen der Salze der 8-Nitronaphthalin-1,3,6-trisulfonsäure mit Wasserstoff unter Druck und in Gegenwart von Katalysatoren gefunden, das dadurch gekennzeichnet ist, daß man als Katalysator Kobalt-, Palladium- oder Platinsulfide verwendet und bei einem pH-Wert von 7,5 bis 12, bei Temperaturen von 30 bis 150°C und 5 bis 150 bar Wasserstoffdruck hydriert.

Dabei kann die Lösung der Salze der 8-Nitro-naphthalin-1,3,6-trisulfonsäure, wie sie bei der Nitrierung der Naphthalin-1,3,6-trisulfonsäure, nach Austreiben der nitrosen Gase und Abtrennen der Schwefelsäure als Gips, technisch anfällt, zur Hydrierung eingesetzt werden. Diese Lösung kann noch die Salze anderer Naphthalinsulfonsäuren und Nitronaphthalinsulfonsäuren als Beimengungen und/oder Verunreinigungen, wie Alkali- und Erdalkalisalze, sowie Eisenverbindungen enthalten. Naphthalin- bzw. Nitronaphthalinsulfonsäuren, die als Beimengungen vorliegen können, sind beispielsweise:

Naphthalin-1,3,6-, Naphthalin-1,3,5-, 1-Nitro-naphthalin-2,5,7-, 1-Nitronaphthalin-4,6,8-, 1-Nitro-naphthalin-3,5,7-, 2-Nitronaphthalin-3,5,7-, 2-Nitronaphthalin-3,6,8- und 2-Nitronaphthalin-4,6,8-trisulfonsäure. Die in das erfindungsgemäße Verfahren einzusetzenden Salze der 8-Nitronaphthalin-1,3,6-trisulfonsäure können beispielsweise bis zu 40% des Gewichtes der Salze der 8-Nitronaphthalin-1,3,6-trisulfonsäure Beimengungen und Verunreinigungen enthalten. Als Verunreinigungen können z.B. Natrium- und Calciumsulfat in Mengen von 0,5 bis 6 Gew.-% und Eisenverbindungen bis zu 0,1 Gew.-% in der Lösung vorliegen.

Bei der erfindungsgemäßen katalytischen Hydrierung werden alle in der wässrigen Lösung enthaltenden Nitronaphthalinsulfonsauren Salze zu den entsprechenden Aminonaphthalinsulfonsauren

2

Salzen reduziert. Es ist ein besonderes Kennzeichen der erfindungsgemäßen Hydrierung, daß in der wässrigen Lösung der 8-Nitro-naphthalin-1,3,6-trisulfonsäure Sulfate und sonstige schwefelhaltigen Verunreinigungen enthalten sein können, die die Lösung tiefbraun färben können. Überraschenderweise wird die erfindungsgemäße Hydrierung durch solche Stoffe nicht inhibiert und der Katalysator nicht vergiftet, wie es gemäß F. Zymalkowski, Katalytische Hydrierungen (1965), S. 34—36 zu erwarten war.

Die wässrigen Lösungen, die der katalytischen Hydrierung unterworfen werden, können bis zu 35 Gew.-% 8-Nitro-naphthalin-1,3,6-trisulfonsaures Salz enthalten. Vorzugsweise werden Lösungen mit 15 bis 30 Gew.-% 8-Nitronaphthalin-1,3,6-trisulfonsäuresalz eingesetzt. Die 8-Nitronaphthalin-1,3,6-trisulfonsäure und die gegebenenfalls anwesenden weiteren Nitronaphthalinsulfonsäuren und/oder Naphthalinsulfonsäuren können beispielsweise als Natrium-, Kalium-, Ammonium- und/oder Calciumsalze vorliegen. Bevorzugt liegt die 8-Nitronaphthalin-1,3,6-trisulfonsäure als Trinatriumsalz vor.

Erfindungsgemäß werden als Katalysatoren Kobalt-, Palladium- und Platinsulfide eingesetzt. Unter Sulfiden sind Mono- und Polysulfide zu verstehen. Bevorzugt wird Kobaltsulfid insbesondere Kobaltpolysulfid als Katalysator verwendet. Das Kobaltpolysulfid kann dabei beispielsweise der Summenformel $CoS_x$ entsprechen, in der x 3 oder 4 oder eine dazwischenliegende Zahl bedeutet.

Die Katalysatoren werden im allgemeinen der Lösung des 8-Nitronaphthalin-1,3,6-trisulfonsäuresalzes zugesetzt. Sie können aber auch vor oder während der Hydrierung in dieser Lösung aus einem nicht-sulfidischen Salz der genannten Metalle, beispielsweise einem Chlorid, Carbonat, Hydroxid, Nitrat, Oxid oder Sulfat oder auch einem handelsüblichen Komplexsalz oder der zugehörigen Säure, wie $H_2(PtCl_6)$ oder $Na_2(PtCl_6)$ und einem Alkali- oder Erdalkalisulfid oder -polysulfid erzeugt werden.

Es ist auch möglich, fein verteiltes Metall, beispielsweise Raney-Kobalt mit Schwefel oder einer Verbindung des zweiwertigen Schwefels außerhalb oder in der zu hydrierenden Lösung zu behandeln und so zu geeigneten Katalysatoren zu kommen. Zur Katalysatorherstellung können debei die Metallverbindungen und die Schwefelverbindungen in einem solchen Verhältnis eingesetzt werden, daß das stöchiometrische Verhältnis von Metall zu Schwefel zwischen 1 und 4, bevorzugt zwischen 2,0 und 3,5 liegt.

Es ist auch möglich, die katalytisch aktiven Komponenten dadurch zu erzeugen, daß auf einem inerten Träger das Metall als Sulfid oder Polysulfid niedergeschlagen oder durch Behandlung von niedergeschlagenem Metall mit Schwefel oder einer geeigneten schwefelhaltigen Verbindung erzeugt wird. Beispielsweise kann Kobaltsulfid verwendet werden, das hergestellt wurde, wie es in der US—PS 2 402 684 beschrieben ist. Eine Vorreduktion mit Wasserstoff ist dabei nicht erforderlich.

Ein katalytisch aktives Metallsulfid kann auch auf geeigneten Trägern aufgebracht werden. Hierzu können die üblichen Träger Verwendung finden, wie sie beispielsweise in Ullmanns Enzyklopädie der technischen Chemie, Band 9 (1957), Seite 263 ff; Houben-Weyl, Methoden der organischen Chemie, Band IV/2 (1955), Seite 147 ff. sowie in Catalysis Vol. 1, Seite 251 ff Reinhold Publ., New York 1954, beschreiben sind. Beispielsweise seien Aktivkohlen, Aluminiumoxide, Siliciumdioxide, Aluminiumsilikate, gegebenenfalls in Verbindungen mit Alkali- und Erdalkali-Verbindungen, wie beispielsweise Spinelle, Titandioxide und Carbide, wie Siliciumcarbide und Wolframcarbide, sowie organische Stoffe wie Seide, Zellstoff und Synthesefasern genannt. Die Katalysatoren können in pulverförmiger, suspendierter, stückiger oder geformter Anordnung Verwendung finden. In einer besonderen Ausführungsform wird eines der oben genannten Metallsulfide auf einem anderen Metallsulfid als Träger verwendet. Als Beispiel hierfür sei $ReS_x$ auf $NiS_x$ als Träger genannt.

Die Menge an katalytisch wirkendem Metallsulfid auf dem Träger kann zwischen 0,1 und 5 Gew.-%, vorzugsweise zwischen 0,5 und 1 Gew.-% betragen.

Die katalytisch aktive Komponente kann in Mengen von 0,005 bis 15 Gew.-%, vorzugsweise 0,05 bis 5 Gew.-%, bezogen auf die Gesamtmenge der Nitroverbindungen eingesetzt werden. Der Katalysator kann für mehrere Hydrierungen verwendet werden. Dabei kann es vorteilhaft sein, den gebrauchten Kontakt vor der Wiederverwendung mit einer Alkalisulfidlösung zu behandeln.

Den Katalysator und die zu hydrierende Lösung kann man auf beliebige Weise zusammenbringen. Bevorzugt wird die zu hydrierende Lösung und der Katalysator gemeinsam in das Reaktionsgefäß eingebracht oder die zu hydrierende Lösung vorgelegt und der Katalysator oder die Katalysator bildenden Lösungen dazu gegeben.

In einer bevorzugten Ausführungsform des Verfahrens läßt man das Metallsulfid erst in der Reduktionslösung entstehen. Ein besonderer Vorteil dieses Verfahrens ist, daß Lösungen eines Metallsalzes einerseits und eine geeignete Sulfidverbindung, wie $Na_2S$, $Na_2S_x$, $NH_4S_x$ oder $NaHS$, unmittelbar der zu hydrierenden Lösung zugeführt werden können. Diese Lösungen können im Gegensatz zu den festen Katalysatoren leicht auch in bereits unter Druck stehende Reaktionsapparate eingepumpt werden, so daß beispielsweise ein kontinuierliches Verfahren, bei dem laufend Katalysatory nachgeführt wird, technisch einfach ausgeführt werden kann.

Zur Durchführung des erfindungsgemäßen Verfahrens kann die Lösung des 8-Nitronaphthalin-1,3,6-trisulfonsäuresalzes in einem üblichen Druckgefäß mit dem Katalysator versetzt und unter 5 bis 150 bar Druck und bei 30 bis 150°C bis zum Ende der Wasserstoffaufnahme hydriert werden. Das Reaktionsgemisch kann anschließend durch Abdekantieren, Zentrifugieren oder Filtrieren vom Katalysator getrennt werden. Der Katalysator kann für weiter Umsetzungen Verwendung finden.

**0 002 691**

Das Verfahren der Erfindung kann technisch unterschiedlich realisiert werden. Es kann beispielsweise als Sumpfphasenhydrierung entsprechend Ullmanns Enzyklopädie der technischen Chemie, Band 10 (1958), Seiten 508 und 560 durchgeführt werden. Hierzu kann die Lösung oder Suspension in Gegenwart des erforderlichen Katalysators durch einen oder mehrere hintereinandergeschalteten Reaktoren geführt werden. Hierbei kann so verfahren werden, daß die Lösung bzw. Suspension mit dem Katalysator durch eine Rührkesselkaskade oder ein System von Röhrenöfen bei den genannten Temperaturen und Drucken gepumpt wird. Der für die Reaktion notwendige Katalysator kann als Frischkatalysator zugeführt werden. Vorteilhaft ist es, den Katalysator wiederzuverwenden, gegebenenfalls unter Zugabe von frischem Katalysator. Beispielsweise kann man so verfahren, daß man nach jedem Durchgang 1 bis 10% des gebrauchten Katalysators abtrennt und durch die entsprechende Menge frischen Katalysator ersetzt.

Das Verfahren kann weiterhin als Rieselphasenhydrierung ausgeführt werden. Dabei kann die Lösung der Nitroverbindungen bei den genannten Temperaturen und Drucken über einen festangeordneten Katalysator geleitet werden. Der Vorteil dieser, beispielsweise in Ullmanns Enzyklopädie der technischen Chemie, Band 7 (1956), Seite 448, beschriebenen Arbeitsweise liegt darin, daß eine Filtration des Katalysators entfällt.

Während der Hydrierung soll der pH—Wert der Lösung bei 7,5 bis 12 liegen. Beispielsweise kann die mit dem Katalysator versetzte Lösung vor der Hydrierung auf einen pH-Wert von 8 bis 10 eingestellt werden. In einer bevorzugten Ausführungsform wird die Lösung des 8-Nitronaphthalin-1,3,6-trisulfonsäuresalzes mit bereits gebrauchtem Katalysator und/oder der wässrigen Lösung eines der genannten Metallsalze und einer wässrigen Natriumpolysulfidlösung versetzt, auf einen pH-Wert von 8 bis 9,5 eingestellt und dann hydriert.

Die Temperatur sollte bei der Hydrierung wenigstens 30°C betragen. Vorzugsweise beträgt die Temperatur 80 bis 135°C. Um kurze Reaktionszeiten zu erzielen kann es vorteilhaft sein, im Temperaturbereich von 110 bis 135°C zu hydrieren.

Der Wasserstoffdruck sollte wenigstens 5 bar betragen. Drucke oberhalb 150 bar sind in der Regel nicht erforderlich. Der Wasserstoffdruck kann während der Hydrierung konstant gehalten werden. Es ist aber auch möglich, den Wasserstoffdruck durch den Verbrauch von Wasserstoff während der Reduktion in einem Druckintervall von wenigen bis zu etwa 100 bar abfallen zu lassen. Bevorzugt hydriert man im Druckbereich von 50 bis 120 bar.

Der für die Reduktion verwendete Wasserstoff kann reiner Wasserstoff sein, z.B. Elektrolytwasserstoff. Ein besonderer Vorteil des Verfahrens ist jedoch, daß auch Wasserstoff verwendet werden kann, der auf Grund von Verunreinigungen wie $H_2S$, $SO_2$, COS oder CO für eine Reduktion von Nitrogruppen in Gegenwart von anderen Katalysatoren nicht geeignet ist.

Die Weiterverarbeitung des nach Abtrennung des Katalysators vorliegenden Gemisches zur Gewinnung von H-Säure kann auf übliche Weise erfolgen, beispielsweise indem man aus dem Gemisch durch Ansäuern die T-Säure abtrennt und mit starkem Alkali die T-Säure in die H-Säure überführt.

Es ist als ausgesprochen überraschend anzusehen, daß bei der erfindungsgemäßen Hydrierung im schwach alkalischen wässrigen Medium, auch bei Temperaturen oberhalb 100°C unter reduzierenden Bedingungen die Abspaltung von Sulfonsäuregruppen praktisch nicht eintritt, da aus Kirk-Othmer, Encyclopedia of Chemical Technology, Vol. 13, S. 716 bekannt ist, daß beim Kochen von 1-Aminonaphthalin-3,6,8-trisulfonsäure mit Zink in verdünnter Natronlauge nicht nur die Nitrogruppe reduziert, sondern auch eine Sulfonsäuregruppe abgespalten und 4-Amino-2,7-naphthalindisulfonsäure (Freund'sche Säure) erhalten wird.

Das erfindungsgemäße Verfahren hat den Vorteil, daß aus den anfallenden Gemischen der Katalysator, beispielsweise durch Filtration, gut abgetrennt werden kann. Weiterhin können die anfallenden Gemische auf einfache Weise aufgearbeitet werden und die Hydrierung der 8-Nitro-naphthalin-1,3,6-trisulfonsäuresalze zu 1-Amino-naphthalin-3,6,8-trisulfonsäuresalzen verläuft mit guten Ausbeuten und Selektivitäten.

## BEISPIELE

### Beispiel 1

In einem Rührautoklaven aus Edelstahl werden 800 g einer Lösung von Natriumsalzen von Nitronaphthalin-trisulfonsäuren mit folgenden Gehalten vorgelegt:

Gehalte in g/100 ml Lösung (bestimmt durch Hochdruck-Flüssigkeits-Chromatographie, gerechnet als freie Säure)

| | |
|---|---|
| 0,08 | Naphthalin-1,3,6-trisulfonsäure |
| 19,68 | 1-Nitronaphthalin-3,6,8-trisulfonsäure |
| 2,66 | 1-Nitronaphthalin-3,5,7-trisulfonsäure |
| 1,11 | 1-Nitronaphthalin-4,6,8-trisulfonsäure |
| 0,56 | 1-Nitronaphthalin-2,5,7-trisulfonsäure |
| 0,31 | 2-Nitronaphthalin-3,5,7-trisulfonsäure |
| 0,17 | 2-Nitronaphthalin-4,6,8-trisulfonsäure |

Der nach Reduktion mit Titan-III-Salz durch Diazotierung gefundene Gehalt an Nitronaphthalinsulfonsäuren (MG 413,3) betrug 21,5%.

Die Lösung wurde unter Rühren mit 16 g wasserhaltigem Kobaltnitrat (Co(NO₃)₂ × 6 H₂O) und 81,6 ml einer Natriumpolysulfidlösung versetzt, die durch Lösen von 13,3 Gew.-Teilen Na₂S × 9 H₂O und 3,4 Gew.-Teilen Schwefel in 83,3 Gew.-Teilen Wasser gewonnen wurde. Durch Zugabe von wässriger Natronlauge zur Gesamtlösung wurde ein pH-Wert von 8,5 eingestellt. Der Autoklav wurde verschlossen und mit Wasserstoff zum Verdrängen der Luft mehrmals gespült. Nach dem Aufheizen auf 120°C wurde Wasserstoff bis zu einem Druck von 80 bar aufgepreßt. 45 Minuten wurde bei 120°C und 60 bis 80 bar Wasserstoffdruck gerührt. Nach dem Abkühlen wurde der Katalysator abfiltriert und für eine weitere Hydrierung von 800 g der Lösung der Nitronaphthalin-trisulfonsäuresalzen eingesetzt. Das Filtrat wurde mit Wasser auf 1600 ml aufgefüllt und analysiert.

Die Diazotierung ergab einen Gehalt an Aminonaphthalintrisulfonsäure (MG 383) von 9,60%; das ist, bezogen auf den durch die Diazotierung bestimmten Gehalt an Nitroverbindungen vor der katalytischen Hydrierung, ein Ausbeute von 96,3%. Die Analyse durch Hochdruck-Flüssigkeits-Chromatographie ergab folgende Zusammensetzung:

| | |
|---|---|
| 0,1 | Gew.-% Naphthalin-1,3,6-trisulfonsäure |
| 8,96 | Gew.-% 1-Amino-naphthalin-3,6,8-trisulfonsäure |
| 1,0 | Gew.-% 1-Amino-naphthalin-3,5,7-trisulfonsäure |
| 0,30 | Gew.-% 1-Amino-naphthalin-2,5,7-trisulfonsäure |
| 0,08 | Gew.-% 2-Amino-naphthalin-3,5,7-trisulfonsäure |
| 0,07 | Gew.-% 2-Amino-naphthalin-4,6,8-trisulfonsäure |

Die Ausbeute an 1-Aminonaphthalin-3,6,8-trisulfonsäure ist nach der Analyse durch Hochdruck-Flüssigkeits-Chromatographie 98,2%.

### Beispiel 2

Der durch Filtration nach einem Versuch gemäß Beispiel 1 zurückgewonnene Katalysator, der 3,2 g Kobalt enthielt, wurde als feuchte Paste mit 200 ml Wasser in einem Rührautoklaven unter Wasserstoffdruck auf 120°C erhitzt und 60 bar Druck mit Wasserstoff eingestellt. In 30 Minuten werden 800 g einer 1-Nitronaphthalin-3,6,8-trisulfonsäure-Lösung eingepumpt.

Gehalte der Lösung in g/100 g Lösung

| | |
|---|---|
| 0,37 | 1-Nitronaphthalin-2,5,7-trisulfonsäure |
| 1,69 | 1-Nitronaphthalin-3,5,7-trisulfonsäure |
| 15,05 | 1-Nitronaphthalin-3,6,8-trisulfonsäure |
| 0,79 | 1-Nitronaphthalin-4,6,8-trisulfonsäure |
| 0,12 | 2-Nitronaphthalin-3,6,8-trisulfonsäure |
| 0,12 | 2-Nitronaphthalin-3,5,7-trisulfonsäure |
| 0,09 | 2-Nitronaphthalin-4,6,8-trisulfonsäure |

18,23

Nach Titan-Reduktion wurde durch Diazotierung ein Gehalt von 19,45 Gew.-% Nitronaphthalinsulfonsäuren (MG 413,3) ermittelt.

Insgesamt 45 Minuten wurde bei 120°C hydriert. Der Druck wurde durch Wasserstoffzugabe bei 60 bar gehalten.

Nach der Hydrierung wurde der Katalysator durch Filtration zurückgewonnen und wie oben beschrieben, für weitere Hydrierungen verwendet. Die hydrierte Lösung der 6. mit dem gleichen Katalysator durchgeführten Reduktion wurde wie in Beispiel 1 auf 1600 ml verdünnt und analysiert:

Zusammensetzung in g/100 ml

| | |
|---|---|
| — | Naphthalin-1,3,6-trisulfonsäure |
| 0,01 | 1-Nitro-naphthalin-3,6,8-trisulfonsäure |
| 0,24 | 1-Aminonaphthalin-2,5,7-trisulfonsäure |
| 0,74 | 1-Aminonaphthalin-3,5,7-trisulfonsäure |
| 7,00 | 1-Aminonaphthalin-3,6,8-trisulfonsäure |
| 0,26 | 1-Aminonaphthalin-4,6,8-trisulfonsäure |
| 0,02 | 2-Aminonaphthalin-3,6,8-trisulfonsäure |
| 0,07 | 2-Aminonaphthalin-3,5,7-trisulfonsäure |
| 0,04 | 2-Aminonaphthalin-4,6,8-trisulfonsäure |
| 0,01 | 1-Aminonaphthalin-3,6-disulfonsäure |

Die Dichte der Lösung war 1059 g/l. Aus der Diazotierung ergab sich ein Gehalt von 8,05 Gew.-%

5

Aminonaphthalintrisulfonsäure (MG 383). Dies entspricht einer Ausbeute an Aminonaphthalinsulfonsäuren von 94,6%.

Die Analyse durch Hochdruck-Flüssigkeits-Chromatographie zeigt, daß bei vollständigem Umsatz der 1-Nitronaphthalin-3,6,8-trisulfonsäure keine Nebenreaktionen abgelaufen sind.

Beispiel 3

800 g der gleichen Lösung wie in Beispiel 2 wurden mit 5 g eines sulfidierten Pt-auf-Kohle-Katalysators (5 Gew.-% Pt, im Handel erhältlich) bei 40°C und 80 bar Wasserstoffdruck hydriert. Die Hydrierzeit betrug 2 Stunden. Die Reduktion hatte das gleiche Ergebnis wie in Beispiel 2.

Beispiel 4

800 g der gleichen Lösung wie in Beispiel 2 wurden mit 5 g sulfidierten Pd-Kohle-Katalysators (5 Gew.-% Pd, im Handel erhältlich) bei 120°C und 80 bar Wasserstoffdruck in 30 Minuten hydriert. Die Reduktion hatte das gleiche Ergebnis wie in Beispiel 2.

**Patentansprüche**

1. Verfahren zur Herstellung von 1-Amino-naphthalin-3,6,8-trisulfonsäure durch Reduktion der alkalischwäßrigen Lösungen der Salze der 8-Nitronaphthalin-1,3,6-trisulfonsäure mit Wasserstoff unter Druck und in Gegenwart von Katalysatoren, dadurch gekennzeichnet, daß man als Katalysator Kobalt-, Palladium- oder Platin-sulfide verwendet und bei einem pH-Wert vopn 7,5 bis 12, bei Temperaturen von 30 bis 150°C und 5 bis 150 bar Wasserstoffdruck hydriert.

2. Verfahren nach Anspruch 1, dadurch gekennzeichnet, daß man als Katalysator Kobaltpolysulfid verwendet.

3. Verfahren nach Anspruch 1 und 2, dadurch gekennzeichnet, daß bei 80 bis 135°C hydriert wird.

4. Verfahren nach Anspruch 1 bis 3, dadurch gekennzeichnet, daß bei einem Wasserstoffdruck von 5 bis 120 bar hydriert wird.

5. Verfahren nach Anspruch 1 bis 4, dadurch gekennzeichnet, daß man Lösungen einsetzt, die bis zu 35 Gew.-% 8-Nitronaphthalin-1,3,6-trisulfonsaures Salz enthalten.

6. Verfahren nach Anspruch 1 bis 5, dadurch gekennzeichnet, daß man Lösungen einsetzt, die neben 8-Nitronaphthalin-1,3,6-trisulfonsaurem Salz noch Salze weiterer Naphthalinsulfonsäuren und Nitronaphthalinsulfonsäuren, sowie Alkali- und Erdalkalisulfate enthalten.

7. Verfahren nach Anspruch 1 bis 6, dadurch gekennzeichnet, daß man das Metallsulfid in der Reduktionslösung entstehen läßt.

8. Verfahren nach Anspruch 1 bis 7, dadurch gekennzeichnet, daß die 8-Nitro-naphthalin-1,3,6-tri-sulfonsäure und die gegebenenfalls anwesenden weiteren Nitronaphthalinsulfonsäuren und/oder Naphthalinsulfonsäuren als Natrium-, Kalium-, Ammonium- und/oder Calciumsalze eingesetzt werden.

**Claims**

1. Process for the preparation of 1-amino-naphthalene-3,6,8-trisulphonic acid by reduction of the alkaline-aqueous solutions of the salts of 8-nitronaphthalene-1,3,6-trisulphonic acid with hydrogen under pressure and in the presence of catalysts, characterised in that cobalt, palladium or platinum sulphides are used as the catalyst and hydrogenation is conducted at a pH value of 7.5 to 12, at temperatures of 30° to 150°C and under a hydrogen pressure of 5 to 150 bars.

2. Process according to Claim 1, characterised in that cobalt polysulphide is used as the catalyst.

3. Process according to Claim 1 and 2, characterised in that the hydrogenation is carried out at 80 to 135°C.

4. Process according to Claim 1 to 3, characterised in that the hydrogenation is carried out under a hydrogen pressure of 5 to 120 bars.

5. Process according to Claim 1 to 4, characterised in that solutions which contain up to 35% by weight of a salt of 8-nitro-naphthalene-1,3,6-trisulphonic acid are employed.

6. Process according to Claim 1 to 5, characterised in that solutions which also contain, in addition to a salt of 8-nitronaphthalene-1,3,6-trisulphonic acid, salts of further naphthalenesulphonic acids and nitronaphthalenesulphonic acids, as well as alkali metal sulphates and alkaline earth metal sulphates, are employed.

7. Process according to Claim 1 to 6, characterised in that the metal sulphide is formed in the reduction solution.

8. Process according to Claim 1 to 7, characterised in that the 8-nitro-naphthalene-1,3,6-tri-sulphonic acid and the further nitronaphthalenesulphonic acids and/or naphthalenesulphonic acids which may be present are employed in the form of sodium salts, potassium salts, ammonium salts and/or calcium salts.

**0 002 691**

Revendications

1. Procédé de préparation de l'acide 1-aminonaphtalène-3,6,8-trisulfonique par réduction de solutions aqueuses alcalines des sels de l'acide 8-nitronaphtalène-1,3,6-trisulfonique par l'hydrogène sous pression et en présence de catalyseurs, ce procédé se caractérisant en ce que l'on utilise en tant que catalyseurs des sulfures de cobalt, de palladium ou de platine, et on hydrogène à un pH de 7,5 à 12, à des températures de 30 à 150°C et des pressions d'hydrogène de 5 à 150 bars.

2. Procédé selon la revendication 1, caractérisé en ce que l'on utilise en tant que catalyseur du polysulfure de cobalt.

3. Procédé selon les revendications 1 et 2, caractérisé en ce que l'on hydrogène à une température de 80 à 135°C.

4. Procédé selon les revendications 1 à 3, caractérisé en ce que l'on hydrogène sous une pression d'hydrogène de 5 à 120 bars.

5. Procédé selon les revendications 1 à 4, caractérisé en ce que l'on part de solutions contenant jusqu'à 35% en poids de sel de l'acide 8-nitronaphtalène-1,3,6-trisulfonique.

6. Procédé selon les revendications 1 à 5, caractérisé en ce que l'on part de solutions contenant en plus d'un sel de l'acide 8-nitronaphtalène-1,3,6-trisulfonique, des sels d'autres acides napthtalène-sulfoniques et nitronaphtalène-sulfoniques, ainsi que des sulfates alcalins et alcalino-terreux.

7. Procédé selon les revendications 1 à 6, caractérisé en ce que l'on forme le sulfure métallique dans la solution soumise à la réduction.

8. Procédé selon les revendications 1 à 7, caractérisé en ce que l'on met l'acide 8-nitronaphtalène-1,3,6-trisulfonique et les autres acides nitronaphtalène-sulfoniques et/ou naphtalène-sulfoniques éventuellement présents en oeuvre à l'état de sels de sodium, de potassium, d'ammonium et/ou de calcium.